# EUROPEAN PATENT SPECIFICATION

(11) **EP 0 445 277 B1**
(45) Date of publication and mention of the grant of the patent: **31.07.1996**
(21) Application number: 90914821.5
(22) Date of filing: 26.09.1990
(51) Int. Cl.: G01N 33/569, G01N 33/574

(54) **QUANTITATIVE IMMUNOCYTOCHEMISTRY ASSAY**
QUANTITATIVE IMMUNOCYTOCHEMISCHE PRÜFUNG
ANALYSE IMMUNOCYTOCHIMIQUE QUANTITATIVE

(30) Priority: 26.09.1989 US 412450
(43) Date of publication of application: 11.09.1991
(73) Proprietor: CITY OF HOPE, Duarte California 91010-0269 (US)
(72) Inventor: BATTIFORA, Hector, A., Arcadia, CA 91006 (US)
(74) Representative: Dowden, Marina
(86) International application number: US9005429
(87) International publication number: WO9105263

(56) References cited:
- US-A- 4 816 410
- LABORATORY INVESTIGATION, vol. 62, no. 1, 1990, Annual Meeting of the Internatl. Academy on Pathology (US-Canadian Div.,Boston, MD (US), 04-09 March 1990; H. BATTIFORA et al., p. 8a, no. 38/
- JOURNAL OF HISTOCHEMISTRY & CYTOCHEMISTRY, vol. 31, no. 1, 1983, US; J. SCHIPPER et al., pp. 12-18/
- JOURNAL OF NEUROSCIENCE METHODS, vol. 26, 1988; L.B. NABORS et al., pp. 25-34/
- AMERICAN JOURNAL OF CLINICAL PATHOLOGY, vol. 90, no. 2, 1988; H.J. HELIN et al., pp. 137-142/
- BIOLOGICAL ABSTRACTS, vol. 77, no. 5, 1984, Biological Abstracts Inc., Philadelphia, PA (US); E.V. GAFFNEY et al., p. 3984, no. 36455/
- PROCEEDINGS OF THE NATL. ACADEMY OF SCIENCES USA, vol. 83, October 1986, Washington, DC (US); W.P. CARNEY et al., pp. 7485-7489/
- WICK et al., "Immunofluorescence Technology-Selected Theoretical and Clinical Aspects", 1982, Elsevier Biomedical Press, Amsterdam (N); pp. 86-90/

## Description

This invention relates to the direct quantitative determination by immunocytochemistry of target molecules in tissue specimens.

L.B. Nabors et al, J. of Neuroscience Methods, **26**:25-34 (1988) refers to methods of establishing the concentration of the amino acid gamma-butyric acid (GABA) from optical density measurements.

US-A-4816410 refers to control slides for use in immunoassays having a section of cell pellet-retained on the slide.

Assays to determine the presence or absence of target molecules in tissue specimens typically entail the running of an appropriate biochemical test on a tissue homogenate. Assays for estrogen receptor (ER) and progesterone receptor (PR) are of value in predicting tumour response and are routinely done in homogenates of breast cancer.

Tissue homogenates for quantitative biochemical assays are based on a variable number of normal cells and cells such as cancer cells which express target molecules. Immunocytochemistry has a theoretical advantage over such biochemical assays in that it directly measures the presence or absence of the target molecules in the relevant cells.

To date, however, this advantage has not been realised because current immunocytochemistry is based on a visual estimation of the intensity of the immunostaining reaction product. This procedure has major drawbacks which preclude its use as a precise, reproducible, quantitative assay. Among other things:
(1) The various tissue processing steps (fixation time, type of fixative, dehydration, embedding and related procedures) are all responsible for some variation, in particular loss of antigen, which modifies the immunostain signal.
   These variables are difficult to control as they depend on fixative concentration, temperature, contaminants and, importantly, time.
(2) The thickness of the tissue section is difficult to control--an important factor because the signal strength increases as a function of section thickness.
(3) The staining procedure will indicate differences in intensity of immunoreactivity depending upon multiple variables, such as antibody concentration, timing, type of chromogen used, and the like.

This invention replaces the visual estimation procedure of the prior art with a practical technique for utilizing the capability of immunocytochemistry to measure quantitatively the presence or absence of a target molecule in the cells of a tissue or other specimen. More particularly, the invention provides a control or internal standard containing a known amount of antigen (or other target molecules) to be processed concurrently with the tissue specimen. The control is thus subjected to all of the same conditions as the tissue specimen to be assayed. For example, a decrease of 30% in the availability of an antigen to be detected will equally affect the control and the specimen under examination. Because the control has cells with a known amount of antigen or other target molecules, a compensating factor can readily be determined, and a quantitative assay of the tissue specimen accomplished.

According to the present invention there is provided a quantitative immunocytochemical assay which comprises:
(i) suspending cells expressing a known, defined amount of a target molecule in an aqueous agar or gelatin gel forming medium;
(ii) causing the suspension to form an agar or gelatin gel having the cells suspended therein;
(iii) determining the optical density of the cells in a section of the gel;
(iv) placing the section of the gel in a tissue cassette together with a specimen of the tissue to be analysed for the target molecule using immunostaining, such that the section and the tissue specimen are concurrently fixed, processed, embedded and stained;
(v) determining the optical density of the fixed processed and embedded cells in the section and in the tissue specimen;
(vi) measuring the difference between the optical density of the cells in the section as determined in step (iii) with the optical density of the cells in the section as determined in step (v) ; and
(vii) utilizing the optical density difference measured in step (vi) to provide a quantitative immunohistochemical determination of the target molecule content of the tissue specimen.

The present invention also provides a quantitative immunocytochemical assay for a target molecule which comprises:
(i) concurrently fixing, processing and embedding a section of an agar or gelatin gel containing cells expressing a known amount of a target molecule and a tissue specimen to be assayed for expression of the target molecule using immunostaining;
(ii) quantitatively determining the difference in the target molecule content of the section of the gel before and after fixing, processing and embedding;
(iii) quantitatively determining the target molecule content of the tissue specimen after concurrent fixing, processing and embedding; and
(iv) utilizing the difference in the target molecule content of the section as determined in step (ii) to provide a quantitative assay of the target molecule content of the tissue specimen to be assayed.

The control provided by this invention may be a section or slice of a medium, such as gelatin, agar or any other aqueous embedding medium, having embedded therein cells, such as MCF7 (a known breast cancer cell line which expresses ER and PR) or BT474 (a known cell line which expresses a breast cancer oncogene), expressing a defined amount of a target molecule. More specifically, cells grown in tissue culture are suspended in the gelatin or other embedding medium which is allowed to solidify by cooling or polymerization. The solidified medium containing the cell suspension is cut into sections or slices of appropriate thickness, e.g., a thickness of from about 2 to about 5 mm.

Choice of cell type depends upon the type of antigen or other molecules to be measured. There is a plethora of cells of many types expressing a variety of antigens or other molecules. Transfected cells may be used. Target molecules which may be quantified in a specimen by use of this invention include, for example, proteins expressed by oncogenes, cell growth factors, and any of the various molecules that control cell proliferation.

The pathologist receiving, for example, a breast biopsy suspected of containing cancer cells includes a control slice embodying the invention into the cassette to ensure that both the tissue sample and the control are concurrently subjected to all ensuing procedures through immunostaining. The optical density of the stained cancer cells in the biopsy sample is compared to that of the cells in the control which acts as an internal standard. Preferably, the comparison is made by use of a computerized cell analysis system, such as a CAS 200 image analyzer (Cell Analysis Systems, Inc., Lombard, Illinois).

### EXAMPLE

Cells grown in tissue culture (MCF7 and BT474), known to express a defined amount of the antigens to be measured, were briefly fixed in paraformaldehyde and suspended in a 3% agar solution (Difco, Detroit, Michigan) at 56°C and allowed to gel. Uniform slices, 3 mm thick, were fixed in formaldehyde for periods of time ranging from 4 to 72 hours and processed together into a single paraffin block. The cross-sectional dimensions of the block were about 2 by about 2.5 cm. Sections cut at 5 µm were immunostained for estrogen receptor (ER-ICA, Abbott, Illinois) and cERB-b2 oncoprotein (Triton Bios., Alameda, California). The intensity of the immunoreactivity was measured for each quantitation of immunocytochemistry gel with a CAS 200 image analyzer. Progressive reduction in the intensity of the immunoreactivity, which correlated with the lengthening of the fixation time was detected with both antigens. Significantly, such reduction was not noticeable by conventional microscopy in the ER-ICA stains.

## Claims

1. A quantitative immunocytochemical assay which comprises:
(i) suspending cells expressing a known, defined amount of a target molecule in an aqueous agar or gelatin gel forming medium;
(ii) causing the suspension to form an agar or gelatin gel having the cells suspended therein;
(iii) determining the optical density of the cells in a section of the gel;
(iv) placing the section of the gel in a tissue cassette together with a specimen of the tissue to be analysed for the target molecule using immunostaining, such that the section and the tissue specimen are concurrently fixed, processed, embedded and stained;
(v) determining the optical density of the fixed, processed, embedded and stained cells in the section and in the tissue specimen;
(vi) measuring the difference between the optical density of the cells as determined in step (iii) with the optical density of the cells as determined in step (v); and
(vii) utilizing the optical density difference measured in step (vi) to provide a quantitative immunohistochemical determination of the target molecule content of the tissue specimen.

2. An assay as claimed in claim 1 in which the target molecule is an estrogen receptor or a progesterone receptor.

3. A quantitative immunocytochemical assay for a target molecule which comprises:
(i) concurrently fixing, processing and embedding
a section of an agar or gelatin gel containing cells expressing a known amount of a target molecule and a tissue specimen to be assayed for expression of the target molecule using immunostaining;
(ii) quantitatively determining the difference in the target molecule content of the section of the gel before and after fixing, processing and embedding;
(iii) quantitatively determining the target molecule content of the tissue specimen after concurrent fixing, processing and embedding; and
(iv) utilizing the difference in the target molecule content of the section as determined in step (ii) to provide a quantitative assay of the target molecule content of the tissue specimen to be assayed.

4. An assay as claimed in claim 3 in which the difference in target molecule content of the section determined in step (ii) is determined by optical density measurement of the cells in the section.

5. An assay as claimed in claim 3 or 4 in which the target molecule is an estrogen receptor or a progesterone receptor.

6. The use of a section of an agar or a gelatin gel having suspended therein cells expressing a known, defined amount of a target molecule in an assay according to any preceding claims.

7. The use of a section as claimed in claim 6 in which the target molecule is a protein expressed by an oncogene, a growth factor, or a receptor in an assay according to any of claims 1 to 5.

8. The use of a section as claimed in claim 6 in which the cells are MCF7 cells or BT474 cells in an assay according to any of claims 1 to 5.

## Patentansprüche

1. Quantitativer immunocytochemischer Assay, der
(i) das Suspendieren von Zellen, die eine bekannte definierte Menge eines Targetmoleküls in einem wäßriges Agar- oder Gelatinegel bildenden Medium exprimieren,
(ii) die Bildung eines Agar- oder Gelatinegels mit den darin suspendierten Zellen in der Suspension,,
(iii) die Bestimmung der optischen Dichte der Zellen in einem Teil des Gels,
(iv) das Einbringen des Teils des Gels in eine Gewebeschale mit einer Probe des auf das Targetmolekül zu analysierenden Gewebes unter Verwendung von Immunfärbung, so daß der Teil und die Gewebeprobe gleichzeitig fixiert, bearbeitet, eingebettet und gefärbt wird,
(v) die Bestimmung der optischen Dichte der fixierten, bearbeiteten, eingebetteten und gefärbten Zellen in dem Teil und in der Gewebeprobe,
(vi) das Messen des Unterschiedes zwischen der optischen Dichte der Zellen, wie sie in Stufe (iii) bestimmt worden sind, mit der optischen Dichte der Zellen, wie sie in der Stufe (v) bestimmt worden sind, und
(vii) die Nutzung der in Stufe (vi) gemessenen optischen Dichtedifferenz zum Bereitstellen einer quantitativen immunohistochemischen Bestimmung des Targetmolekülgehalts der Gewebeprobe
umfaßt.

2. Assay nach Anspruch 1, in dem das Targetmolekül ein Östrogenrezeptor oder eine Progesteronrezeptor ist.

3. Quantitativer immunocytochemischer Assay für ein Targetmolekül, der
(i) das gleichzeitige Fixieren, Bearbeiten und Einbetten eines Teiles eines Agar- oder Geleatinegels der Zellen, die eine bekannte Menge eines Targetmoleküls und einer zu bestimmenden Gewebeprobe zum Exprimieren des Targetmoleküls unter Verwendung von Immunofärbung enthält,
(ii) das quantitative Bestimmen der Differenz in dem Targetmolekülgehalt des Teils des Gels vor und nach dem Fixieren, Bearbeiten und Einbetten,
(iii) das quantitative Bestimmen des Targetmolekülgehalts der Gewebeprobe nach dem gleichzeitigen Fixieren, Bearbeiten und Einbetten und
(iv) das Nutzen der Differenz im Targetmolekülgehalt des Teils, wie sie in Stufe (ii) bestimmt worden ist, um einen quantitativen Assay des Targetmolekülgehalts der zu prüfenden Probe bereitzustellen,
umfaßt.

4. Assay nach Anspruch 3, in dem der Unterschied im Targetmolekülgehalt des in Stufe (ii) bestimmten Teils durch optische Dichtemessung der Zellen in dem Teil bestimmt wird.

5. Assay nach Anspruch 3 oder 4, in dem das Targetmolekül ein Östrogenrezeptor oder ein Progesteronrezeptor ist.

6. Verwendung eines Teils eines Agar- oder Gelatinegels mit darin suspendierten eine bekannte definierte Menge eines Targetmoleküls exprimierenden Zellen in einem Assay nach einem der vorhergehenden Ansprüche.

7. Verwendung eines Teils nach Anspruch 6 in einem Assay nach einem der Ansprüche 1 bis 5, in dem das Targetmolekül ein durch ein Onkogen, einen Wachstumsfaktor oder einen Rezeptor exprimiertes Protein ist.

8. Verwendung eines Teils nach Anspruch 6 in einem Assay nach einem der Ansprüche 1 bis 5, in dem die Zellen MCF7-Zellen oder BT474-Zellen sind.

## Revendications

1. Analyse immunocytochimique quantitative, qui comprend les étapes consistant :
(i) à mettre en suspension des cellules exprimant une quantité connue et définie d'une molécule cible dans un milieu aqueux formant de la gélose ou un gel de gélatine ;
(ii) à faire en sorte que la suspension forme une gélose ou un gel de gélatine dans lequel les cellules sont en suspension ;
(iii) à déterminer la densité optique des cellules dans une coupe du gel ;
(iv) à placer la coupe du gel dans une cassette de tissu en même temps qu'un échantillon du tissu dont on veut analyser la molécule cible par utilisation d'une immunocoloration, de façon que la coupe et l'échantillon de tissu soient simultanément fixés, traités, incorporés et colorés ;
(v) à déterminer la densité optique des cellules fixées, traitées, incorporées et colorées, dans la coupe et dans l'échantillon de tissu ;
(vi) à mesurer la différence entre la densité optique des cellules telle que déterminée dans l'étape (iii) et la densité optique des cellules telle que déterminée dans l'étape (v) ; et
(vii) à utiliser la différence de densité optique mesurée dans l'étape (vi) pour réaliser une détermination immunohistochimique quantitative de la teneur en molécule cible du spécimen de tissu.

2. Analyse selon la revendication 1, dans laquelle la molécule cible est un récepteur d'oestrogène ou un récepteur de progestérone.

3. Analyse immunocytochimique quantitative portant sur une molécule cible, qui comprend les étapes consistant :
(i) simultanément, à fixer, traiter et incorporer une coupe d'une gélose ou d'un gel de gélatine contenant des cellules exprimant une quantité connue d'une molécule cible et un échantillon de tissu dont on veut déterminer l'expression de la molécule cible, par immunocoloration ;
(ii) à déterminer d'une manière quantitative la différence de teneur en molécule cible de la coupe du gel avant et après fixation, traitement et incorporation ;
(iii) à déterminer d'une manière quantitative la teneur en molécule cible de l'échantillon de tissu après les opérations simultanées de fixation, de traitement et d'incorporation ; et
(iv) à utiliser la différence de teneur en molécule cible de la coupe, telle que déterminée dans l'étape (ii), pour fournir une analyse quantitative de la teneur en molécule cible de l'échantillon de tissu à analyser.

4. Analyse selon la revendication 3, dans laquelle la différence de teneur en molécule cible, de la coupe, déterminée dans l'étape (ii), est déterminée par une mesure de la densité optique des cellules dans la coupe.

5. Analyse selon la revendication 3 ou 4, dans laquelle la molécule cible est un récepteur d'oestrogène ou un récepteur de progestérone.

6. Utilisation d'une coupe d'une gélose ou d'un gel de gélatine dans lequel sont suspendues des cellules exprimant une quantité connue et définie d'une molécule cible, dans le cadre d'une analyse selon l'une quelconque des revendications précédentes.

7. Utilisation d'une coupe selon la revendication 6, dans laquelle la molécule cible est une protéine exprimée par un oncogène, un facteur de croissance ou un récepteur, dans le cadre d'une analyse selon l'une quelconque des revendications 1 à 5.

8. Utilisation d'une coupe selon la revendication 6, dans laquelle les cellules sont des cellules MCF7 ou des cellules BT474 dans le cadre d'une analyse selon l'une quelconque des revendications 1 à 5.
